# EUROPÄISCHE PATENTSCHRIFT

(11) **EP 2 274 019 B1**
(45) Veröffentlichungstag und Bekanntmachung des Hinweises auf die Patenterteilung: **19.09.2012**
(21) Anmeldenummer: 09801410.3
(22) Anmeldetag: 18.12.2009
(51) Int. Cl.: A61K 49/00, A61M 5/178

(54) **FARBSTOFFLÖSUNG**
DYE SOLUTION
SOLUTION COLORANTE

(30) Priorität: 19.12.2008 DE 102008064065
(43) Veröffentlichungstag der Anmeldung: 19.01.2011
(73) Patentinhaber: FLUORON GMBH, 89077 Ulm (DE)
(72) Erfinder: LINGENFELDER, Christian, Dr., 89081 Ulm (DE); THEISINGER, Bastian, 68229 Mannheim (DE); HIEBL, Wilfried, Dr., 89257 Illertissen (DE); HAGEDORN, Nadine, 89134 Blaustein (DE)
(74) Vertreter: Schiweck, Weinzierl & Koch
(86) Internationale Anmeldenummer: PCT/EP2009/009144
(87) Internationale Veröffentlichungsnummer: WO 2010/078942

(56) Entgegenhaltungen:
- WO-A1-2004/035091
- WO-A1-2010/060018
- WO-A2-03/057259
- ÜNLÜ K ET AL.: "Gentian violet solution for staining the anterior capsule" J CATARACT REFRACT SURG, Bd. 26, 1. August 2000 (2000-08-01), Seiten 1228-11232, XP002605866 & Alcon Laboratories: "BSS Plus"[Online] Gefunden im Internet: URL:http://ecatalog.alcon.com/pi/BSSplus25 0_us_en.pdf> [gefunden am 2010-10-08]

## Beschreibung

### Gebiet der Erfindung

Die Erfindung betrifft eine wasserbasierte biokompatible Zubereitung zum selektiven Anfärben von Membrana limitans interna (ILM) und/oder von epiretinalen Membranen (EMR) im menschlichen oder tierischen Auge und ein Kit enthaltend die besagte erfindungsgemäße wasserbasierte biokompatible Zubereitung.

### Hintergrund der Erfindung

Erkrankungen des Auges, wie Grauer Star (Katarakt), Glaukom (Grüner Star), altersbedingte Makuladegeneration und Diabetes-bedingte Retinopathie, sowie Netzhautveränderungen bzw. Netzhautablösungen nehmen zu, bedingt unter anderem durch die höhere Lebenserwartung. Zu der Behandlung dieser und weiterer Augenkrankheiten ist häufig eine Vitrektomie indiziert. Dabei muss sichergestellt werden, dass die Netzhaut möglichst wenig geschädigt wird. Eine Vorsichtsmaßnahme besteht darin, bei der Vitrektomie die Membrana limitans interna (ILM) und ggf. epiretinale Membranen von der Netzhaut zu entfernen, um die Makula von den vermuteten intravitrealen Zugkräften zu entlasten. Dazu werden die Membranen mit einer Pinzette von der Netzhaut abgezogen. Für den Chirurgen ist es notwendig, dass er möglichst genau zwischen Netzhaut und abzuziehender Membran unterscheiden kann. Dazu sollen die abzuziehenden Membranen über eine möglichst spezifische Anfärbung sichtbar gemacht werden. Für eine Anfärbung geeignete Farbstoffe müssen viele Kriterien erfüllen. Sie müssen biokompatibel und untoxisch sein und dürfen die Zellen nicht schädigen, sie sollten wasserlöslich sein, sie sollten möglichst spezifisch anfärben und leicht wieder ausspülbar sein. Es wurden bereits Farbstoffe und Verfahren zur Anfärbung der genannten Membranen beschrieben, die aber noch nicht voll befriedigen.

So beschreibt US 7,014,991 ein Verfahren zur Anfärbung von okularen Strukturen im menschlichen Auge, wobei das Anfärben durch Einspritzen des Farbstoffes Indigotindisulfat in das entsprechende Gewebe erfolgt. Indigotindisulfat ist jedoch cytotoxisch.

Weitere Farbstoffe, wie Brillantblau G, Brilliant Blau R, Patentblau V, oder Methylenblau wurden ebenfalls zur Verwendung im Auge vorgeschlagen. EP 1553 984 beschreibt Triphenylmethanfarbstoffe, z.B. BBR und Patentblau V zum Anfärben von aus dem Auge zu entfernenden Membranen. Ünlü et al. (2000), J. Cataract Refract. Surg. beschreibt Enzianblau zum selektiven Anfärben der vorderen Augenkammer.

Während der Vitrektomie oder des chirurgischen Eingriffs wird die Augenhöhle mit einer Spüllösung gespült. Ein Problem der bisher bekannten Farbstofflösungen besteht nun darin, dass durch die Spüllösung die Farbstofflösung verteilt, verdünnt und ausgespült wird. Dies hat mehrere Nachteile. Zum einen wird die Sicht des Chirurgen getrübt, wenn die Spüllösung gefärbt ist. Zum anderen ist mehr Farbstofflösung erforderlich, als nur zum Anfärben der Membran notwendig wäre.

Um diesen Nachteil zu überwinden, wurde schon vorgeschlagen, der Farbstofflösung ein Verdickungsmittel, wie z.B. Hyaluronsäure, zuzusetzen, das die Viskosität der Farbstofflösung erhöht. Die Erhöhung der Viskosität soll dazu führen, dass der Farbstoff aufgrund verminderter Beweglichkeit, also durch sterische Hinderung, nicht so stark in die Spüllösung übertritt und eher in die Nähe der anzufärbenden Membran gelangt. Allerdings führt die hohe Viskosität der Farbstofflösung dazu, dass der Farbstoff nunmehr aus dieser heraus schlecht auf die Membran übertreten kann, so das wiederum der Bedarf an Farbstofflösung höher ist als die Menge, die nur zur Anfärbung der Membran notwendig wäre. WO 03/057259 beschreibt eine ICG-Zubereitung aus Alkohol oder Polyvinylpyrrolidon.

Es war daher Aufgabe der Erfindung, eine Zubereitung bereitzustellen, die Membranen speziell anfärben kann, insbesondere die zu entfernenden Membranen wie Membrana limitans interna (ILM) und/oder epiretinale Membranen (ERM), im menschlichen oder tierischen Auge selektiv anfärben kann, die sich gut applizieren lässt, direkt nach der Applikation zur Membran wandert und sich dort verteilt, ohne die Spüllösung zu stark anzufärben. Weiterhin soll eine Zubereitung bereitgestellt werden, die weder zu lokalen Reizungen noch Schädigungen der Netzhaut führt, nicht cytotoxisch ist, sondern gut vertragen wird.

Diese Aufgabe wird gelöst mit einer Zubereitung, wie sie in den Ansprüchen, insbesondere in Anspruch 1 definiert ist.

Die Unteransprüche enthalten vorteilhafte Welterbildungen.

### Detaillierte Beschreibung der Erfindung

Überraschenderweise wurde gefunden, dass eine Zubereitung, die mindestens Brillantblau G als Farbstoff enthält, dann ein effektives und selektives Anfärben der ILM und/oder der ERM zulässt, wenn die Dichte der Zubereitung in einem Bereich von 1,01 g/cm³ bis 1,5 g/cm³, bevorzugt 1,01 g/cm³ bis 1,3 g/cm³ eingestellt wird.

Es wurde gefunden, dass eine Farbstofflösung mit gegenüber dem Wasser erhöhter Dichte dann, wenn sie im Rahmen einer chirurgischen Behandlung am Auge in den Bereich der Augenhöhle eingespritzt wird, absinkt, wodurch ein schnelles Vermischen mit der Spüllösung vermieden wird, und sich nach dem Absinken auf der Membran verteilt und diese anfärbt. Dadurch wird vermieden, dass der Farbstoff mit der Spüllösung zu schnell weggespült wird und auch, dass der Farbstoff das Sichtfeld eintrübt.

Die erfindungsgemäße Zubereitung basiert auf Wasser als Lösungsmittel, wobei weitere Lösungsmittel gegebenenfalls in geringeren Anteilen enthalten sein können, sofern sie mit Wasser homogen vermischbar sind und biologisch kompatibel sind. In Betracht kommen hier ein- und mehrwertige Alkohole, wie sie im medizinischen Bereich auch Anwendung finden. Falls ein weiteres Lösungsmittel verwendet wird, ist dieses besonders bevorzugt ein Glycol oder Glycerin. Auch Mischungen der genannten Lösungsmittel kommen in Betracht. Falls dem Wasser ein Lösungsmittel zugemischt wird, so sollte dieses in einem Anteil von nicht mehr als 20 Gew.-%, bevorzugter nicht mehr als 10 Gew.-% verwendet werden. Bevorzugt ist die Zubereitung einer istotonischen Lösung.

Außer Wasser als Lösungsmittel und dem Farbstoff, der unten näher ausgeführt wird, enthält die erfindungsgemäße Zubereitung als wesentlichen Bestandteil ein die Dichte einstellendes Mittel. Das die Dichte einstellende Mittel muss biologisch kompatibel sein, darf nicht toxisch sein und muss mit Wasser homogen vermischbar sein, ggf. nach Zugabe einer geringen Menge eines Löslichkeitsvermittlers wie Alkohol, so dass eine klare transparente Lösung entsteht. Außerdem muss sie mit dem Farbstoff kompatibel sein, d.h. darf nicht die Löslichkeit des Farbstoffs in erheblichem Ausmaß beeinträchtigen. Bei der Einstellung der Zubereitung ist auch die Osmolarität zu beachten, um keine durch Osmose bedingten Schäden am Gewebe hervorzurufen. Die Osmolarität sollte in einem Bereich von 280-330 mosmol/L, bevorzugt bei 300 mosmol/L liegen.

In Betracht kommen daher mit Wasser kompatible Flüssigkeiten, deren Dichte über der Dichte des Wassers liegt. Ein vorteilhaftes Mittel zur Erhöhung der Dichte ist schweres Wasser, D₂O, mit dem der Dichtewert auf den gewünschten Bereich eingestellt werden kann. Schweres Wasser zeichnet sich durch eine hervorragende Verträglichkeit aus. Es wird bis zu einer Konzentration von 20 % in Wasser von Eukaryoten toleriert und führt nicht zu Reizungen im Anwendungsgebiet. Es ist mit Wasser in jeder beliebigen Konzentration mischbar, neigt nicht zum Absetzen oder zur Separation und weist bezüglich der Löslichkeit keine nachweisbaren Unterschiede gegenüber Wasser auf. Der Anteil an schwerem Wasser kann in der Zubereitung so eingestellt werden, dass der gewünschte Dichtewert von 1,01 g/cm³ bis 1,5 g/cm³, bevorzugt von 1,01 g/cm³ bis 1,3 g/cm³, erreicht wird. Die geeignete Menge, die auch von den weiteren Inhaltsstoffen abhängt, kann durch einfache Versuche oder Berechnungen gefunden werden. Wenn schweres Wasser das die Dichte einstellende Mittel ist, wird es bevorzugt in einer Menge von 5-20% verwendet. Auch die Herstellung der Zubereitung mit schwerem Wasser ist sehr einfach und kann einfach durch Vermischen erfolgen aufgrund der guten Vermischbarkeit der beiden Bestandteile. Aus Wasser, schwerem Wasser und Farbstoff lässt sich daher einfach und schnell eine auf Dauer stabile und für den Zweck des selektiven Anfärbens der Membranen gut geeignete Zubereitung herstellen.

Ein weiteres Mittel, mit dem die Dichte eingestellt werden kann, ist ein Di- oder Polysaccharid. Polysaccharide eignen sich zur Erhöhung der Dichte und sind gut verfügbar. Außerdem sind sie toxikologisch unbedenklich und biologisch kompatibel. Unter Polysacchariden werden hier Moleküle verstanden, die aus mehr als zwei, bevorzugt mehr als 5, besonders bevorzugt mehr als 10 Saccharideinheiten aufgebaut sind. Obwohl allgemein Mono- und Disaccharide die Dichte erhöhen können, werden erfindungsgemäß nur nicht-reduzierende Disaccharide zur Erhöhung der Dichte eingesetzt. Die Verwendung von Monosacchariden und reduzierenden Disacchariden kann zu unerwünschten Wirkungen führen, so können diese z.B. in der zur Erhöhung der Dichte notwendigen Menge cytotoxisch sein. Erfindungsgemäß geeignete nicht-reduzierende Disaccharide sind Saccharose oder Trehalose. Als geeignete Polysaccharide können lösliche Stärkederivate, wie Hydroxyethylstärke und Dextran genannt werden. Als Polysaccharide sind solche Verbindungen geeignet, die neutral sind, nicht reduzierend wirken und sich in wässriger Lösung nicht abbauen.

Weitere Mittel zur Einstellung der Dichte sind neutrale Polymere wie Polyether, Polyvinylalkohol, Polyester, Polyacrylsäure Copolymer, Polyvinylpyrrolidon.

Auch Kombinationen der genannten Mittel sind gut geeignet, um die Dichte der erfindungsgemäßen Zubereitung einzustellen, z.B. eine Kombination aus schwerem Wasser und einem oder mehreren Polysacchariden.

Die Menge an schwerem Wasser und/oder weiteren oder anderen die Dichte einstellenden Mitteln wird dabei so gewählt, dass die Dichte der fertig hergestellten Zubereitung im erforderlichen Bereich von 1,01 g/cm³ bis 1,5 g/cm³, bevorzugt 1,01 g/cm³ bis 1,3 g/cm³ liegt. Die Dichte der Zubereitung kann dabei nach jeder gängigen Methode bestimmt werden, wie sie dem Fachmann allgemein bekannt ist.

Es hat sich herausgestellt, dass die Erhöhung der Dichte auf 1,01 g/cm³ bereits den gewünschten Effekt bewirkt, nämlich dass die Farbstofflösung nach Applikation in die Augenhöhle schnell nach unten sinkt und sich dort dann auf der Membran verteilen kann. Dadurch gelingt ein selektives Anfärben der Membran, ohne die Sicht des Operateurs zu verschlechtern. Ein Dichteunterschied von weniger als 0,01 g/cm³ bezogen auf Wasser ist nicht mehr ausreichend, um die Farbstoffzubereitung gezielt absinken zu lassen. Das Absinken erfolgt dann so langsam wie bei den Zubereitungen des Standes der Technik und führt zu den oben aufgeführten Problemen. Wenn die Dichte der Zubereitung größer als 1,5 g/cm³ ist, so kann es aufgrund der Dichte zu einer Schädigung der sehr empfindlichen Netzhaut kommen.

Ein weiterer wichtiger Aspekt der erfindungsgemäßen Zubereitung ist Brillantblau G als Farbstoff. Als Farbstoff können solche Verbindungen eingesetzt werden, die die ILM und/oder EMR spezifisch und gezielt anfärben können, so dass sich die Membran optisch von der Netzhaut unterscheidet. Weiterhin muss der Farbstoff in dem Wasser bzw. der Mischung aus Wasser und einem weiteren Lösungsmittel löslich sein. Er darf weder toxisch, insbesondere cytotoxisch bzw. zellschädigend sein, noch Schäden an der Netzhaut hervorrufen oder durch Lichtreaktionen toxische Wirkungen entwickeln wie ICG oder Trypanblau. Außerdem sollte er ein gutes Färbevermögen haben, um die Menge des Farbstoffs gering halten zu können.

Als vorteilhaft haben sich Farbstoffe aus der Gruppe der Triphenylmethanfarbstoffe, wie Brillantblau G, Brilliant Blau R, Brilliant Blau FCF, Patentblau V, Bromphenolblau, Lissamine Grün SF, Lissamine Grün G, Fast Green, Methylgrün, Brilliantsäuregrün, Commassie Violet R 200, Rosanillin; aus der Gruppe der Azo- bzw. Diazofarbstoffe wie Orange G, Ponceau 2R, Chromotrope 6 R, Ponceau 6 R, Tartrazine, Azophloxine, Ponceau B, Evans-Blau, Chicago Blau; aus der Gruppe der Cyaninfarbstoffe wie 3,3'-Diethylthiacyanine iodide, 3,3'-Diethylthiacarbocyanine iodide, 3,3'-Diethyl-9-methylthiacarbocyanine iodide, 1,1'-Diethyl-4,4'-cyanine iodide und/oder aus der Gruppe der Naturfarbstoffe wie Orcein, Lawsone, Indigotin, Canthaxanthin, Hematoxylin, Indigocarmine und/oder Anthocyanen und Anthocyanidinen sowie deren Mischungen, d.h. Mischungen aus mehreren Mitgliedern einer der genannten Gruppen als auch aus Mitgliedern unterschiedlicher Gruppen, erwiesen.

Als weitere Farbstoffe könnten Brilliantblau R, Brilliantblau FCF, Patentblau V, Methylgrün, Commassie Violet R 200, Bromphenolblau und/oder Chicagoblau verwendet werden. Von den Triphenylmethanfarbstoffen könnten Coomassie Violet R 200 und Chicagoblau besonders bevorzugt verwendet werden. Von den Brillantblau-Farbstoffen ist Brilliantblau G aufgrund seines besonders guten Färbevermögens bevorzugt. Er kann in einer Konzentration von weniger als 0,3 g/l eingesetzt werden. Diese geringe Konzentration führt bereits zu einer ausreichenden selektiven Färbung von ILM und/oder EMR. Weitere geeignete Farbstoffe sind Lissamine Grün SF, Lissamine Grün G, Fast Green, Brilliantsäuregrün, Orange G, Ponceau 2R, Chromotrope 6 R, Ponceau 6 R, Tartrazine, Azophloxine, Ponceau B, Chicago Blau, Evans-Blau, 3,3'-Diethylthlacyanine iodide, 3,3'-Diethylthiacarbocyanine iodide, 3,3'- Diethyl-9-methylthlacarbocyanine iodide, 1,1'- Diethyl-4,4'-cyanine iodide, Orcein, Lawsone, Indigotin, Canthaxanthin, Hematoxylin, Indigocarmine und verschiedene Anthocyane.

Um die vorteilhaften Eigenschaften der erfindungsgemäßen Zubereitung weiter zu verbessern, kann der Zubereitung zusätzlich noch ein die Viskosität einstellendes Mittel zugefügt werden. Es hat sich gezeigt, dass der Zusatz eines die Viskosität der erfindungsgemäßen Zubereitung erhöhenden Mittels eine Verbesserung der Kohäsivität bewirken kann, sodass die mit der erfindungsgemäßen Zubereitung erhaltenen Vorteile noch verstärkt werden. Die applizierte Zubereitung, die aufgrund der höheren Dichte schneller absinkt, wird noch weniger in der Spüllösung verteilt, da sie aufgrund der erhöhten Viskosität bis zum Auftreffen auf die Membran zusammengehalten wird. Da ein vorteilhafter Effekt aber bereits durch Einstellung der Dichte erreicht wird, muss die Viskosität nicht so stark erhöht werden, dass dies zu Problemen führt, wie sie im Stand der Technik bestehen. Bereits eine geringe Anhebung der Viskosität führt dazu, dass die aus dem Applikationsgerät austretenden Tropfen eine stabilere Einheit bilden und damit weniger leicht verdünnbar sind, was verhindert, dass der in der Zubereitung eingebettete Farbstoff ausgeschwemmt wird. Dadurch wird der Farbstoff erst am Applikationsort durch Kapillareffekte auf der Membran freigesetzt, die sich dadurch einfärbt. Auf diese Weise kann der Farbstoff gezielt zur Membran gebracht werden.

Als viskositätsregulierende biokompatible Mittel, also Mittel, die die Viskosität einstellen, können aus der folgenden Gruppe ein oder mehrere eingesetzt werden: Polyether, Polyvinylalkohol, Polyester, Polyacrylsäure Copolymer, Polyvinylpyrrolidon, und andere Polymere, mehrwertige Alkohole wie Glycerin, Propylenglycol, Butylenglycol, wasserlösliche Cellulosederivate wie Methylcellulose, Xanthan Gummi, Stärke, Hyaluronsäure und deren jeweilige Derivate, Chondroitinsulfat und Natriumsulfat. Als die Viskosität regulierende Mittel können auch solche verwendet werden, die nicht nur die Viskosität, sondern gleichzeitig auch die Dichte erhöhen. In diesem Fall ist es wichtig, darauf zu achten, dass beide Parameter, d.h. sowohl die Viskosität als auch die Dichte, im gewünschten Bereich liegen. Mit anderen Worten darf ein die Dichte beeinflussendes und die Viskosität regulierendes Mittel nicht in einer solchen Menge eingesetzt werden, dass die fertige Zubereitung dann eine Dichte von mehr als 1,5 g/cm³ aufweist. Die geeigneten Mengen können jedoch vom Fachmann leicht, gegebenenfalls mit Routineversuchen, festgestellt und die entsprechenden Werte in der Zubereitung eingestellt werden.

Besonders geeignet als viskositätsregulierende Mittel sind solche, die eine gewisse Affinität zu dem erfindungsgemäß verwendeten Farbstoff aufweisen und sich durch ein hohes Spreitvermögen auszeichnen. Überraschenderweise wurde gefunden, dass Butylenglycol ein Mittel ist, mit dem die Viskosität reguliert werden kann und das zu einem guten Spreitvermögen führt. Ein Zusatz von Butylenglycol kann daher dafür sorgen, dass die applizierte Zubereitung nach unten sinkt und, sobald sie die Membran erreicht hat, sich dort ausbreitet und die Membran schnell anfärbt. Dies wird, ohne an eine Theorie gebunden zu sein, damit erklärt, dass einerseits Butylenglycol eine Affinität zu Membranen hat und andererseits aufgrund lipophiler Gruppen den Farbstoff gut adsorbiert. Wenn die butylenglycol- und farbstoffhaltige Zubereitung die Membran erreicht, sorgt das Butylenglycol dafür, dass sich der Farbstoff schnell auf der Membran verteilen kann

Die Viskosität der erfindungsgemäßen Zubereitung wird bevorzugt so eingestellt, dass die Scherviskosität bei 25 °C und einer Scherrate von 10 s⁻¹ in einem Bereich vom 1 bis 500 mPas liegt. Bevorzugt wird die Scherviskosität bei 25 °C und einer Scherrate von 10 s⁻¹ auf einen Bereich von 50 bis 275 mPas eingestellt. Das Einstellen der Viskosität kann mit den bereits genannten viskositätsregulierenden Mitteln erzielt werden. Liegt die Viskosität, unter den angegebenen Messbedingungen in einem Bereich von 1 bis 500 mPas, so werden die mit der erfindungsgemäßen Zubereitung erzielten Effekte deutlich verstärkt. Die den selektiv färbenden Farbstoff enthaltende Zubereitung sinkt schnell ab, ohne dass der Farbstoff in nennenswertem Umfang mit der Spüllösung ausgewaschen wird. Der Farbstoff wird somit erst am Applikationsort durch Kapillareffekte auf der Membran freigesetzt, die sich dadurch einfärbt. Ist die Viskosität unter den angegebenen Messbedingungen geringer als 1 mPas, so kann der Effekt des schnellen Absetzens der erfindungsgemäßen Zubereitung nicht zusätzlich verstärkt werden. Es besteht die Möglichkeit, dass wenigstens ein Teil des Farbstoffes vor dem Anfärben der Membran mit der Spüllösung ausgetragen wird und somit nicht mehr für das Anfärben der Membran zur Verfügung steht. Liegt die dynamische Viskosität bei 25 °C und einer Scherrate von 10 s⁻¹ dagegen oberhalb von 500 mPas, so ist die Viskosität der Zubereitung so hoch, dass der Farbstoff nicht optimal aus den sich bildenden Tröpfchen freigesetzt werden kann. Die Fähigkeit der Farbstoffzubereitung zu spreiten, was eine schnelle, homogene Anfärbung der Membran bewirkt, wird dadurch deutlich reduziert. Die Membran wird nicht optimal mit der Farbstoffzubereitung benetzt und damit auch nicht so deutlich gefärbt. Ein besonders gutes Anfärbeergebnis wird erreicht, wenn die dynamische Viskosität bei 25 °C und einer Scherrate von 10 s⁻¹ in einem Bereich von 50 bis 275 mPas liegt.

Es wurde gefunden, dass bei Verabreichung von Farbstofflösungen in das Auge Probleme auftreten können. Wenn die Farbstofflösung mit den üblicherweise verwendeten Spritzen verabreicht wird, wird der Druck, der beim Einspritzen erzielt wird, zu hoch, so dass der Farbstoff hinter die Netzhaut geraten kann.

Das Problem wird erfindungsgemäß gelöst, indem Spritzen verwendet werden, bei denen Kanülendurchmesser, das Verhältnis von Zylinderdurchmesser zu Kanüfendurchmesser und das Aspektverhältnis so angepasst sind, dass Schäden vermieden werden. Erfindungsgemäß werden bevorzugt Spritzen verwendet, bei denen der Kanülendurchmesser sehr klein ist, um die Schädigung im Auge möglichst gering zu halten. Weiterhin wird der Zylinderdurchmesser so an den Kanülendurchmesser angepasst, dass das Auftreten eines Venturi-Effekts weitgehend vermieden wird. Mit anderen Worten muss bei der zur Verabreichung vorgesehenen Spritze auch der Durchmesser des Zylinders möglichst klein sein, sodass das Verhältnis von Zylinderdurchmesser zu Kanülendurchmesser im Bereich von 10 bis 2 zu 1 bis 0,2, bevorzugt 20 zu1 bis 4 zu 1, besonders bevorzugt 16 zu 1 bis 8 zu 1 liegt. Außerdem sollten die Spritzenzylinder ein Aspektverhältnis, d.h. Verhältnis von Zylinderlänge zu Zylinderdurchmesser, in einem Bereich von 15 bis 5 zu 1 haben.

Ein weiterer Gegenstand der Erfindung ist daher ein Kit, der eine Spritze mit Zylinder und Kanüle enthaltend eine erfindungsgemäße Farbstoffzubereitung zum selektiven Anfärben der Membrana limitans interna und/oder von epiretinalen Membranen im menschlichen oder tierischen Auge, umfasst, wobei das Verhältnis von Zylinderdurchmesser zu Kanülendurchmesser im Bereich von 10 - 2 zu 1 - 0,2, bevorzugt 20:1 bis 4:1, besonders bevorzugt 16:1 bis 8:1 liegt. Bevorzugt ist das Verhältnis von Zylinderlänge zu Zylinderdurchmesser in einem Bereich von 15 bis 5 zu 1. Als erfindungswesentlichen Bestandteil weist der Kit somit eine Spritze auf, deren Zylinderdurchmesser an den Durchmesser der Kanüle angepasst ist. Es wurde gefunden, dass bei einem kleineren Verhältnis der Durchmesser sich im Innenraum vor der Kanüle kein Druck aufbauen kann, so dass ein gleichmäßiges Applizieren, also ein Applizieren mit gleichmäßigem Druck und gleichbleibender Geschwindigkeit der erfindungsgemäßen Zubereitung gewährleistet ist. Der Kit eine erfindungsgemäße Farbstoffzubereitung wie oben beschrieben.

Für den Kit, bzw. dessen Spritze wird bevorzugt eine Kanüle mit 19 bis 27 gauge, besonders bevorzugt 23 oder 25 gauge verwendet. Kanülen mit 19 bis 27 gauge sind für Injektionen in das Auge geeignet. Ihre Austrittsöffnung ist so klein, dass sie keine nennenswerten Schäden am Injektionsort hinterlassen, aber dennoch groß genug, um die erfindungsgemäße Zubereitung im Auge mit einer ausreichenden Geschwindigkeit zu applizieren. Wenn der Zylinder der Spritze im Durchmesser entsprechend angepasst ist, wird vermieden, dass sich im Inneren der Spritze bzw. der Kanüle ein Druck aufbaut, der bei der Injektion die Zubereitung unter zu starkem Druck in das Auge bringt. so dass die Zubereitung über den Applikationsort hinaus, z.B. hinter der Netzhaut, verteilt wird. Als besonders gut hinsichtlich der erwünschten Applikation haben sich Kanülen mit 20, 23, 25 oder 27 gauge, insbesondere solche mit 23 oder 25 gauge erwiesen. In einer bevorzugten Ausführungsform wird eine derartige Kanüle zusammen mit einer Spritze mit einem Zylinderdurchmesser von 3 bis 10 mm eingesetzt. Besonders bevorzugt sind Kanülen mit 23 oder 25 gauge, wenn die dynamische Viskosität der Zubereitung bei 25 °C und einer Scherrate von 10⁻¹ in einem Bereich von 1 bis 500 mPas liegt. Gerade dann ist das Zusammenspiel zwischen Kanüle und Zubereitung so gut, dass in ausreichend schneller Geschwindigkeit gleichmäßig eine genügend große Menge der erfindungsgemäßen Zubereitung am Applikationsort abgesetzt werden kann, ohne dass es durch Druckstauung zum explosionsartigen Austritt der Zubereitung aus der Kanüle kommt. Somit wird verhindert, dass die Zubereitung hinter den gewünschten Applikationsort injiziert wird, wodurch ein optimales Anfärben der Membran erzielt werden kann.

Die oben beschriebenen erfindungsgemäßen Zubereitungen und die zu ihrer Verabreichung bereitgestellten Spritzen ermöglichen es, im Auge gezielt Membranen - ILM und/oder ERM - anzufärben. Je nach eingesetztem Farbstoff ist es möglich entweder nur eine Art von Membran, d.h. nur ILM oder nur ERM anzufärben, oder aber beide Arten anzufärben. In einer Ausführungsform kann die erfindungsgemäße Zubereitung dazu verwendet werden, eine Negativfärbung der epiretinalen Membranen zu bewirken, um diese dann entfernen zu können. In dieser Ausführungsform wird eine Lösung des Farbstoffs Brillant Blau G, eingesetzt, der selektiv die ILM, nicht aber die ERM färbt. Auf diese Weise lässt sich die nicht gefärbte Membran (ERM) von der gefärbten Membran (ILM) unterscheiden und kann daher gut entfernt werden.

Die Erfindung wird noch durch die folgenden Beispiele, die Farbstofflösungen mit erhöhter Dichte und deren Herstellung beschreiben, erläutert, ohne sie darauf zu beschränken.

### Beispiel 1

0,025 g Brilliantblau G, 5 g Saccharose, 0,19 g Dinatriumhydrogenphosphat, 0,03 g Natriumdihydrogenphosphat und 0,82 g Natriumchlorid werden genau eingewogen und mit destilliertem Waser auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L und einer Dichte von 1,023 g/cm³ entsteht.

### Beispiel 2

0,025 g Brilliantblau G, 5 g Trehalose , 0,19 g Dinatriumhydrogenphosphat, 0,03 g Natriumdihydrogenphosphat und 0,82 g Natriumchlorid werden genau eingewogen und mit destilliertem Waser auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L und einer Dichte von 1,023 g/cm³ entsteht.

### Beispiel 3

0,025 g Brilliantblau G, 0,19 g Dinatriumhydrogenphosphat, 0,03 g Natriumdihydrogenphosphat und 0,82 g Natriumchlorid werden genau eingewogen und mit einer Mischung aus destilliertem Wasser und D₂O auf 100 g aufgefüllt. Die Rohstoffe werden in einer Glasflasche für 1 h bei maximal 60 °C behandelt, wobei eine homogene Lösung mit einer Farbstoffkonzentration von 0,25 g/L, einer Dichte von 1,018 g/cm³ entsteht.

### Beispiel 4

### Farbstoff+Glycerin

0,025 g Brilliantblau G, 0,19 g Dinatriumhydrogenphosphat, 0,03 g Natriumdihydrogenphosphat und 0,82 g Natriumchlorid werden genau eingewogen und mit einer Mischung aus destilliertem Wasser und 10 % Glycerin aufgefüllt. Die Rohstoffe werden in einer Glasflasche für 1 h bei maximal 60 °C behandelt, wobei eine homogene Lösung mit einer Farbstoffkonzentration von 0,25 g/L, einer Dichte von 1,027 g/cm³.

### Beispiel 5

Mit dem Verfahren, wie in den Beispielen 1 bis 4 beschrieben wurde eine Farbstofflösung mit folgender Zusammensetzung hergestellt

| Substanz | Einwaage soll ist in g | Einwaage ist in g |
|---|---|---|
| Polyvinylpyyrolidon | 6 | 6,0067 |
| Brilliant Blau G | 0,0125 | 0,0125 |
| Na₂HPO₄*2H₂O | 0,095 | 0,0950 |
| NaH₂PO₄*2H₂O | 0,015 | 0,0159 |
| NaCl | 0,41 | 0,4100 |
| Wasser | auf 50 g | auf 50 g |

Es wurde eine homogene Lösung mit einer Dichte von 1,028 g/cm³ und einer Viskosität von 7,38 mPas erhalten.

### Beispiel 6

Mit dem Verfahren, wie in den Beispielen 1 bis 4 beschrieben wurde eine Farbstofflösung mit folgender Zusammensetzung hergestellt

| Substanz | Einwaage soll ist in g | Einwaage ist in g |
|---|---|---|
| Methylcellulose E 10 M (2 wt.%) | 25 | 24,9986 |
| Brilliant Blau G | 0,0125 | 0,0125 |
| Na₂HPO₄*2H₂O | 0,095 | 0,0956 |
| NaH₂PO₄*2H₂O | 0,015 | 0,0151 |
| NaCl | 0,41 | 0,4099 |
| Wasser | auf 50 g | auf 50 g |

Es wurde eine homogene Lösung mit einer Dichte von 1,007 g/cm³ und einer Viskosität von 142,79 mPas erhalten.

Die in den Beispielen 1 bis 6 hergestellten Farbstofflösungen wurden zur Anfärbung der Membrana limitans interna im menschlichen oder tierischen Auge verwendet. Es wurde gefunden, dass sich alle sechs Lösungen sehr gut auftragen ließen und nach der Applikation sofort absanken und die ILM anfärbten Die Färbung war, bei gleicher Menge an Farbstoff, noch intensiver als mit einer zum Vergleich aufgetragenen Briliant Blau G Lösung, wie sie aus DE 10255601 bekannt ist.

## Patentansprüche

1. Wasserbasierte, biokompatible Zubereitung zum selektiven Anfärben der Membrana limitans interna (ILM) und/oder von epiretinalen Membranen (ERM) im menschlichen oder tierischen Auge, enthaltend mindestens Brillantblau G als Farbstoff, wobei die Zubereitung eine Dichte im Bereich von 1,01 g/cm³ bis 1,5 g/cm³ aufweist.

2. Zubereitung nach Anspruch 1, zusätzlich enthaltend einen Farbstoff ausgewählt aus der Gruppen bestehend aus Azofarbstoffen, Cyaninfarbstoffen und/oder Naturfarbstoffen oder deren Mischungen.

3. Zubereitung nach Anspruch 1 oder 2, wobei die Zubereitung eine Dichte im Bereich von 1,01 g/cm³ bis 1,3 g/cm³ aufweist.

4. Zubereitung nach einem der Ansprüche 1 bis 3, **dadurch gekennzeichnet, dass** die Konzentration des Brillantblau G in der fertigen Zubereitung bis zu 0,3 g/l, bevorzugt 0,25 g/L beträgt.

5. Zubereitung nach einem der vorhergehenden Ansprüche zur Verwendung als Farbstoff für eine Negativdarstellung epiretinaler Membranen.

6. Zubereitung nach einem der vorhergehenden. Ansprüche, **dadurch gekennzeichnet, dass** sie als ein die Dichte der Zubereitung einstellendes Mittel schweres Wasser, ein Polymer ausgewählt aus Polyether, Polyvinylalkohol, Polyester, Polyacrylsäure-Copolymer, Polyvinylpyrrolidon, ein Polysaccharid, ausgewählt aus Hydroxyethylstärke oder Dextran, oder Mischungen davon enthält.

7. Zubereitung nach einem der Ansprüche 1 bis 5, **dadurch gekennzeichnet, dass** sie als ein die Dichte der Zubereitung einstellendes Mittel einen Polyether enthält.

8. Zubereitung nach einem der vorhergehenden Ansprüche, **dadurch gekennzeichnet, dass** sie zusätzlich mindestens ein die Viskosität einstellendes Mittel enthält, ausgewählt aus: Polyether, Polyvinylalkohol, Polyester, Polyacrylsäure Copolymer, Polyvinylpyrrolidon, und anderen Polymeren, mehrwertigen Alkoholen wie Glycerin, Ethylenglycol, Propylenglycol, Butylenglycol, Cellulose, Xanthan Gummi, Stärke, Hyaluronsäure und deren jeweilige Derivate, Chondroitinsulfat und Natriumsulfat.

9. Zubereitung nach einem der Ansprüche 1 bis 8, **dadurch gekennzeichnet, dass** die Zubereitung bei 25°C und einer Scherrate von 10 s⁻¹ eine dynamische Viskosität im Bereich von 1 bis 500 mPas, bevorzugt in einem Bereich von 50 bis 275 mPas auf.

10. Zubereitung nach Anspruch 1 bis 9 **dadurch gekennzeichnet, dass** die Osmolarität der hergestellten Lösungen im Bereich von 280-330 mosmol/L, bevorzugt 300 mosmol/L, liegt.

11. Kit umfassend eine Spritze mit Zylinder und Kanüle und eine wasserbasierte Zubereitung gemäß einem der Ansprüche 1 bis 10 zum selektiven Anfärben der Membrana limitans interna (ILM) und/oder von epiretinalen Membranen (ERM) im menschlichen oder tierischen Auge, wobei das Verhältnis von Zylinderdurchmesser zu Kanülendurchmesser von 10 bis 2 zu 1 bis 0,2, bevorzugt 20 zu1 bis 4 zu 1 beträgt.

12. Kit nach Anspruch 11, **dadurch gekennzeichnet dass** das Verhältnis von Zylinderlänge zu Zylinderdurchmesser in einem Bereich von 15 bis 5 zu 1 liegt.

13. Kit nach einem Anspruch 11 oder 12, **dadurch gekennzeichnet, dass** die Spritze eine Kanüle mit 19 bis 27 gauge, bevorzugt 23 oder 25 gauge, aufweist.

14. Kit nach Anspruch 13, **dadurch gekennzeichnet, dass** die Spritze einen Zylinderdurchmesser von 3 bis 10 mm hat.

15. Zubereitung nach einem der Ansprüche 1 bis 10 oder Kit nach einem der Ansprüche 11 bis 14 zur Verwendung bei der chirurgischen Behandlung am Auge, umfassend das selektive Anfärben der Membrana limitans interna (ILM) und/oder von epiretinalen Membranen (ERM) im menschlichen oder tierischen Auge.

## Claims

1. A water-based, biocompatible preparation for the selective staining of the internal limiting membrane (ILM) and/or of epiretinal membranes (ERM) in the human or animal eye, containing at least Brilliant Blue G as dye, wherein the preparation has a density in the range of 1.01 g/cm³ to 1.5 g/cm³.

2. The preparation according to claim 1, additionally containing a dye selected from the group consisting of azo dyes, cyanine dyes and/or natural dyes or mixtures thereof.

3. The preparation according to claim 1 or 2, wherein the preparation has a density in the range of 1.01 g/cm³ to 1.3 g/cm³.

4. The preparation according to one of claims 1 to 3, **characterised in that** the concentration of the Brilliant Blue G in the finished preparation is up to 0.3 g/l, preferably 0.25 g/l.

5. The preparation according to one of the preceding claims for use as a dye for a negative representation of epiretinal membranes.

6. The preparation according to one of the preceding claims, **characterised in that**, as an agent that adjusts the density of the preparation, it contains heavy water, a polymer selected from polyethers, polyvinyl alcohol, polyesters, polyacrylic acid copolymers, polyvinyl pyrrolidone, a polysaccharide selected from hydroxyethyl starch or dextran, or mixtures thereof.

7. The preparation according to one of claims 1 to 5, **characterised in that**, as an agent that adjusts the density of the preparation, it contains a polyether.

8. The preparation according to one of the preceding claims, **characterised in that** it additionally contains at least one viscosity-adjusting agent, selected from: polyethers, polyvinyl alcohol, polyesters, polyacrylic acid copolymers, polyvinyl pyrrolidone, and other polymers, polyhydric alcohols such as glycerol, ethylene glycol, propylene glycol, butylene glycol, cellulose, xanthan gum, starch, hyaluronic acid and the respective derivatives thereof, chondroitin sulfate and sodium sulfate.

9. The preparation according to one of claims 1 to 8, **characterised in that** the preparation has a dynamic viscosity in the range of 1 to 500 mPas, preferably in a range of 50 to 275 mPas, at 25°C and a shear rate of 10 s⁻¹.

10. The preparation according to claims 1 to 9, **characterised in that** the osmolarity of the solutions produced is in the range of 280-330 mosmol/l, preferably 300 mosmol/l.

11. A kit comprising a syringe with barrel and cannula and a water-based preparation according to one of claims 1 to 10 for the selective staining of the internal limiting membrane (ILM) and/or of epiretinal membranes (ERM) in the human or animal eye, wherein the ratio of barrel diameter to cannula diameter is from 10 to 2 : 1 to 0.2, preferably 20 : 1 to 4 : 1.

12. The kit according to claim 11, **characterised in that** the ratio of barrel length to barrel diameter is in a range of 15 to 5 : 1.

13. The kit according to claim 11 or 12, **characterised in that** the syringe has a cannula with 19 to 27 gauge, preferably 23 or 25 gauge.

14. The kit according to claim 13, **characterised in that** the syringe has a barrel diameter of 3 to 10 mm.

15. The preparation according to one of claims 1 to 10 or the kit according to one of claims 11 to 14 for use in surgical treatment on the eye, comprising the selective staining of the internal limiting membrane (ILM) and/or of epiretinal membranes (ERM) in the human or animal eye.

## Revendications

1. Préparation biocompatible aqueuse pour la coloration sélective de la membrane limitante interne (ILM) et/ou de membranes épirétiniennes (ERM) dans l'oeil humain ou animal, contenant au moins du bleu brillant G en tant que colorant, dans laquelle la préparation présente une densité dans la plage de 1,01 g/cm³ à 1,5 g/cm³.

2. Préparation selon la revendication 1, contenant en outre un colorant sélectionné parmi les groupes constitués de colorants azoïques, colorants de cyanine et/ou colorants naturels ou leurs mélanges.

3. Préparation selon la revendication 1 ou 2, dans laquelle la préparation présente une densité dans la plage de 1,01 g/cm³ à 1,3 g/cm³.

4. Préparation selon l'une quelconque des revendications 1 à 3, **caractérisée en ce que** la concentration du bleu brillant G dans la préparation finie va jusqu'à 0,3 g/l, de préférence 0,25 g/l.

5. Préparation selon l'une quelconque des revendications précédentes pour l'utilisation en tant que colorant pour une représentation négative de membranes épirétiniennes.

6. Préparation selon l'une quelconque des revendications précédentes, **caractérisée en ce qu'**elle contient en tant qu'agent réglant la densité de la préparation de l'eau lourde, un polymère sélectionné parmi le polyéther, l'alcool polyvinylique, le polyester, un copolymère d'acide polyacrylique, la polyvinylpyrrolidone, un polysaccharide sélectionné parmi l'amidon hydroxyéthylique ou le dextrane, ou des mélanges de ceux-ci.

7. Préparation selon l'une quelconque des revendications 1 à 5, **caractérisée en ce qu'**elle contient en tant qu'agent réglant la densité de la préparation un polyéther.

8. Préparation selon l'une quelconque des revendications précédentes, **caractérisée en ce qu'**elle contient en outre au moins un agent réglant la viscosité sélectionné parmi : le polyéther, l'alcool polyvinylique, le polyester, un copolymère d'acide polyacrylique, la polyvinylpyrrolidone et d'autres polymères, des alcools polyvalents comme la glycérine, l'éthylèneglycol, le propylèneglycol, le butylèneglycol, la cellulose, la gomme xanthane, l'amidon, l'acide hyaluronique et leurs dérivés respectifs, le sulfate de chondroïtine et le sulfate de sodium.

9. Préparation selon l'une quelconque des revendications 1 à 8, **caractérisée en ce que** la préparation présente à 25°C et à une vitesse de cisaillement de 10 s⁻¹ une viscosité dynamique dans la plage de 1 à 500 mPas, de préférence dans une plage de 50 à 275 mPas.

10. Préparation selon les revendications 1 à 9, **caractérisée en ce que** l'osmolarité des solutions fabriquées se situe dans la plage de 280 à 330 mosmol/l, de préférence à 300 mosmol/l.

11. Kit comprenant une seringue avec cylindre et canule et une préparation aqueuse selon l'une quelconque des revendications 1 à 10 pour la coloration sélective de la membrane limitante interne (ILM) et/ou de membranes épirétiniennes (ERM) dans l'oeil humain ou animal, dans lequel le rapport entre diamètre du cylindre et diamètre de la canule va de 10 à 2 sur 1 à 0,2, de préférence 20 sur 1 à 4 sur 1.

12. Kit selon la revendication 11, **caractérisé en ce que** le rapport entre longueur du cylindre et diamètre du cylindre se situe dans une plage de 15 à 5 sur 1.

13. Kit selon la revendication 11 ou 12, **caractérisé en ce que** la seringue présente une canule avec un calibre de 19 à 27, de préférence un calibre de 23 ou 25.

14. Kit selon la revendication 13, **caractérisé en ce que** la seringue a un diamètre du cylindre de 3 à 10 mm.

15. Préparation selon l'une quelconque des revendications 1 à 10 ou kit selon l'une quelconque des revendications 11 à 14 pour l'utilisation lors du traitement chirurgical sur l'oeil, comprenant la coloration sélective de la membrane limitante interne (ILM) et/ou de membranes épirétiniennes (ERM) dans l'oeil humain ou animal.
